(19) Europäisches Patentamt
European Patent Office
Office européen des brevets

(11) Veröffentlichungsnummer: **0 156 195**
**B1**

(12) # EUROPÄISCHE PATENTSCHRIFT

(45) Veröffentlichungstag der Patentschrift: **11.05.88**

(51) Int. Cl.⁴: **C 07 D 319/20**

(21) Anmeldenummer: **85102438.0**

(22) Anmeldetag: **05.03.85**

(54) **Verfahren zur Herstellung von 2,2,3,3-Tetrachlor-1,4-benzodioxenen und die neue Verbindung 6-Isocyanato-2,2,3,3-tetrachlor-1,4-benzodioxen.**

(30) Priorität: **15.03.84 DE 3409438**

(43) Veröffentlichungstag der Anmeldung: **02.10.85 Patentblatt 85/40**

(45) Bekanntmachung des Hinweises auf die Patenterteilung: **11.05.88 Patentblatt 88/19**

(84) Benannte Vertragsstaaten: **CH DE FR GB IT LI**

(56) Entgegenhaltungen:
**DE - A - 2 848 531**

**CHEMICAL ABSTRACTS, Band 77, Nr. 1, 3. Juli 1972, Columbus, Ohio, USA, MAULDING, D.R.; Roberts, B.C. "Chemiluminescence of tetrachloroethylene carbonate and related compounds", Seite 423, Zusammenfassung Nr. 4708z**
**CHEMICAL ABSTRACTS, Band 89, Nr. 13, 25. September 1978, Columbus, Ohio, USA, G. COUDERT, G. GUILLAUMET, B. LOUBINOUX "Synthesis of 1,4-benzodioxins", Seite 902, Zusammenfassung Nr. 109 290h**

(73) Patentinhaber: **BAYER AG, Konzernverwaltung RP Patentabteilung, D-5090 Leverkusen 1 Bayerwerk (DE)**

(72) Erfinder: **Marhold, Albrecht, Dr., Carl-Duisberg-Strasse 329, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Aus Tetrahedron Letters 1978 (12), Seiten 1059–62 ist ein Verfahren zur Herstellung von 2,3-Dibrom-1,4-benzodioxenen und von im nicht-aromatischen Molekülteil unsubstituierten oder mit einer $CO_2Et$-Gruppe substituierten 1,4-Benzodioxinen bekannt. Aus dieser Literaturstelle kann die vorliegende Erfindung, die ein Tetrachlorbenzodioxen und ein Herstellungsverfahren für Tetrachlorbenzodioxene betrifft, nicht hergeleitet werden.

Es wurde ein Verfahren zur Herstellung von 2, 2, 3, 3-Tetrachlor-1,4-benzodioxenen der Formel

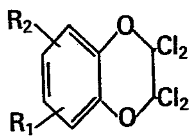

(I),

in der $R_1$ und $R_2$ unabhängig voneinander für H, Cl, F, $CF_3$, $CCl_3$, COCl, CN, $OCOCF_3$, $OCOCCl_3$, $OCOCF_2Cl$, OCOCl, OCOF, NCO, Phenyl oder mit einem oder mehreren der vorstehenden Reste substituiertes Phenyl stehen, gefunden, dass dadurch gekennzeichnet ist, dass man eine oder mehrere Verbindungen der Formel

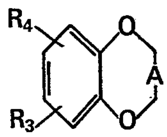

(II),

in der $R_3$ und $R_4$ unabhängig voneinander für H, Cl, F, $CF_3$, $CCl_3$, COCl, CN, $OCOCF_3$, $OCOCCl_3$, $OCOCF_2Cl$, OCOCl, OCOF, NCO, CHO, $CH_3$, Phenyl oder mit einem oder mehreren der vorstehenden Reste substituiertes Phenyl stehen und

A für $CH_2$-$CH_2$, $CHCl$-$CH_2$, $CCl_2$-$CH_2$, CHCl-CHCl, $CCl_2$-CHCl, CH=CH, CCl=CH oder CCl=CCl,

mit Chlor in Gegenwart eines Radikalbildners umsetzt.

Die Ausgangsprodukte der Formel (II) sind beispielsweise gemäss dem in R.C. Elderfield, Heterocyclic Compounds, Vol. 6, Seite 69 beschriebenen Verfahren oder auf dazu analoge Weise zugänglich.

Vorzugsweise werden in das erfindungsgemässe Verfahren solche Verbindungen der Formel (II) eingesetzt, bei denen $R_3$ und $R_4$ unabhängig voneinander für H, Cl, F, COCl, CN, $CH_3$ oder CHO stehen.

Weiterhin ist der Einsatz von solchen Verbindungen der Formel (II) bevorzugt, bei denen A für $CH_2$-$CH_2$, $CCl_2$-$CH_2$, CHCl-CHCl oder CH=CH steht.

Besonders bevorzugt werden Verbindungen der Formel (II) eingesetzt, bei denen $R_3$ und $R_4$ für H und A für $CH_2$-$CH_2$, $CCl_2$-$CH_2$ oder CCl=CH oder $R_3$ für $CH_3$ oder CHO, $R_4$ für H und A für $CH_2$-$CH_2$ stehen.

Werden Verbindungen der Formel (II) eingesetzt, bei denen $R_3$ und/oder $R_4$ für CHO, $CH_3$ oder einen CHO und/oder $CH_3$ und $CH_3$ enthaltenden Rest stehen, so werden die Reste CHO und $CH_3$ im allgemeinen ebenfalls chloriert. Dabei entsteht aus CHO eine COCl- und aus $CH_3$ eine $CCl_3$-Gruppe.

Von den Verbindungen der Formel (II) können in dass erfindungsgemässe Verfahren einzelne Verbindungen, aber auch beliebige Mischungen dieser Verbindungen mit zwei oder mehr Komponenten eingesetzt werden. Von den Mischungen aus Verbindungen der Formel (II) sind zum Einsatz in das erfindungsgemässe Verfahren solche bevorzugt, bei denen sich die einzelnen Komponenten nur hinsichtlich des Molekülteiles A unterscheiden, hinsichtlich der Molekülteile $R_1$ und $R_2$ aber identisch sind, da nur dann einheitliche Reaktionsprodukte erhalten werden können.

In das erfindungsgemässe Verfahren kann Chlorgas in technischen Qualitäten eingesetzt werden, wie sie für Chlorierungsreaktionen üblicherweise verwendet werden. Im allgemeinen ist es vorteilhaft, das Chlor im Überschuss einzusetzen. Beispielsweise kann man pro Mol einzuführendes Chlor 1 bis 5 Mole $Cl_2$ und zusätzlich pro Mol abzuspaltenden Wasserstoff, falls solcher vorhanden ist, weitere 1 bis 5 Mole $Cl_2$ einsetzen. Vorzugsweise setzt man pro Mol einzuführendes Chlor 1,2 bis 2,8 Mole $Cl_2$ und zusätzlich pro Mol abzuspaltenden Wasserstoff, falls solcher vorhanden ist, weitere 1,2 bis 2,8 Mole $Cl_2$ ein.

Wenn in das erfindungsgemässe Verfahren Verbindungen der Formel (II) eingesetzt werden, in denen A für $CH_2$–$CH_2$ oder CH=CH steht, ist es vorteilhaft, das Chlor zunächst so zuzugeben, dass im Reaktionsgemisch eine Chlorkonzentration von 0,5 bis 1 Gew.-% nicht überschritten wird. Wenn sich aus den Verbindungen der Formel (II), in denen A für $CH_2$-$CH_2$ steht das entsprechende Monochlor-1,4-benzodioxen und aus Verbindungen, in denen A für CH=CH steht, das entsprechende Dichlor-1,4-benzodioxen gebildet hat oder Verbindungen der Formel (II), in denen A für CHCl-$CH_2$, $CCl_2$-$CH_2$, CHCl-CHCl, $CCl_2$CHCl, CCl=CH oder CCl=CCl steht, eingesetzt werden, kann die Chlorierung in üblicher Weise ohne Kontrolle der Chlorkonzentration im Reaktionsgemisch durchgeführt werden.

Die Messung der Chlorkonzentration im Reaktionsgemisch kann auf übliche Weise erfolgen, z.B. durch Bestimmung der Extinktion (siehe Houben-Weyl, Methoden der organischen Chemie, Band IV/5 a, Seiten 102–103 (1975)). Eine grobe Abschätzung, ob die Chlorkonzentration unter 0,5 bis 1 Gew.-% liegt, ist durch die Beobachtung der Farbe des Reaktionsgemisches möglich, da die grünlich-gelbe Farbe des Chlors ungefähr in diesem Konzentrationsbereich beginnt sichtbar zu werden. Der Fortgang der Chlorierung, z.B. die Bildung von Monochlor-1,4-benzodioxenen aus Verbindungen der Formel (II), in denen A für $CH_2$-$CH_2$ oder die Bildung von Dichlor-1,4-benzodioxenen aus Verbindungen der Formel (II), in denen A für CH=CH steht, kann man auf einfache Weise verfolgen, beispielsweise durch Bestimmung der Dichte oder durch gaschromatographische Analyse.

Als in das erfindungsgemässe Verfahren einzusetzende Radikalbildner kommen beispielsweise

die für radikalische Chlorierungsreaktionen üblichen Radikalbildner in Frage (siehe z.B. Houben-Weyl, a.a.O.). Bevorzugt ist der Einsatz von UV-Licht, Azoisobuttersäurenitril (ABN) und Benzoylperoxyd. ABN und Benzoylperoxid können dabei in üblichen Zubereitungen und Mengen eingesetzt werden.

Das erfindungsgemässe Verfahren kann mit und ohne Zusatz von Lösungsmitteln durchgeführt werden. Vorzugsweise wird ohne Lösungsmittel oder in konzentrierter Lösung, beispielsweise in 20 bis 80 gew.-%iger Lösung gearbeitet, wenn Verbindungen der Formel (II) eingesetzt werden, bei denen $R_3$ und/oder $R_4$ für Cl, F, $CF_3$, $CCl_3$ oder COCl und/oder A für $CHCl\text{-}CH_2$, $CCl_2\text{-}CH_2$, CHCl-CHCl, $CCl_2\text{-}CHCl$, CCl=CH oder CCl=CCl stehen. Die erfindungsgemässe Umsetzung mit Verbindungen der Formel (II), bei denen $R_3$ und/oder $R_4$ für H oder NCO und/oder A für $CH_2\text{-}CH_2$ oder CH=CH stehen, erfolgt vorzugsweise in verdünnter Lösung, beispielsweise in einer 5 bis 20 gew.-%iger Lösung. Geeignete Lösungsmittel sind beispielsweise Tetrachlormethan, Difluortetrachlorethan, Phosphoroxychlorid und Dichlorbenzol. Vorzugsweise wird Tetrachlormethan oder Phosphoroxychlorid als Lösungsmittel verwendet.

Das erfindungsgemässe Verfahren kann im allgemeinen bei Normaldruck und bei Temperaturen von beispielsweise 25 bis 180°C durchgeführt werden. Bevorzugt sind Temperaturen im Bereich von 80 bis 130°C. Sofern ein Lösungsmittel eingesetzt wird, ist es vorteilhaft am Siedepunkt des Lösungsmittels zu arbeiten. Das erfindungsgemässe Verfahren kann kontinuierlich oder diskontinuierlich durchgeführt werden.

Die Aufarbeitung des nach Durchführung des erfindungsgemässen Verfahrens vorliegenden Reaktionsgemisches kann beispielsweise so erfolgen, dass man zunächst zur Entfernung des überschüssigen Chlors und des gegebenenfalls gebildeten Chlorwasserstoffs einen Inertgasstrom, z.B. Stickstoff, durch das Reaktionsgemisch leitet, anschliessend, soweit vorhanden, das Lösungsmittel abtrennt, z.B. durch Destillation, gegebenenfalls unter vermindertem Druck, und das gebildete 2, 2, 3, 3-Tetrachlor-1,4-benzodioxen durch Destillation im Vakuum isoliert.

Bei einer beispielhaften Ausführungsform des erfindungsgemässen Verfahrens wird wie folgt gearbeitet:

Die Lösung einer Verbindung der Formel (II) in Tetrachlormethan oder Phosphoroxychlorid wird auf Rückflusstemperatur erhitzt. Dann wird das Reaktionsgemisch mit UV-Licht bestrahlt oder ein Radikalbildner zugesetzt und Chlorgas eingeleitet. In den weiter oben beschriebenen Fällen wird zu Beginn der Reaktion die Konzentration von Chlor im Reaktionsgemisch unter 0,5 bis 1 Gew.-% gehalten. Wenn das Reaktionsgemisch kein Chlor mehr aufnimmt, wird die Chlorzufuhr unterbrochen, Stickstoff durch das Reaktionsgemisch geleitet und anschliessend zunächst das Lösungsmittel und dann das gebildete 2, 2, 2, 3-Tetrachlor-1,4-benzodioxen der Formel (I) durch Destillation im Vakuum abgetrennt.

Die nach dem erfindungsgemässen Verfahren herstellbaren 2, 2, 3, 3-Tetrachlor-1,4-benzodioxene der Formel (I) sind wertvolle Produkte, aus denen sich z.B. durch eine an sich bekannte Fluorierung mit Fluorwasserstoff und gegebenenfalls anschliessende Nitrierung und Reduktion Aniline herstellen lassen, aus denen durch Umsetzung mit Acylisocyanaten Verbindungen zugänglich sind, die gemäss der DE-OS'en 3 023 328 und 3 223 505 eine überlegene insektizide Wirkung zeigen. Damit können diese Verbindungen mit überlegener insektizider Wirkung aus besser zugänglichen Ausgangsstoffen und auf einfachere Weise als bisher, sowie in guten Ausbeuten und Reinheiten hergestellt werden.

Überraschenderweise erhält man nach dem erfindungsgemässen Verfahren die 2, 2, 3, 3-Tetrachlor-1,4-benzodioxene der Formel (I) in hohen Selektivitäten und Ausbeuten. Eine an sich zu erwartende Kernchlorierung tritt allenfalls nur in geringen Anteilen auf.

Die vorliegende Erfindung betrifft weiterhin die neue Verbindung 6-Isocyanato-2,2,3,3-tetrachlor-1,4-benzodioxen der Formel

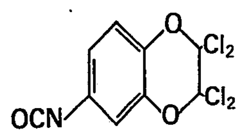

(III).

Die Herstellung dieser Verbindung kann wie vorstehend beschrieben, ausgehend z.B. von 6-Isocyanato-1,4-benzodioxen (siehe Formel (II), $R_3$=NCO in 6-Stellung, $R_4$=H, A=$CH_2\text{-}CH_2$), erfolgen. Die Verwendbarkeit dieser Verbindung ist ebenfalls vorstehend beschrieben.

Die folgenden Beispiele erläutern die Erfindung ohne sie in irgendeiner Weise zu beschränken.

Beispiele:

Allgemeine Versuchsbeschreibung:

Die folgenden Beispiele wurden in einer Chlorierungsapparatur durchgeführt, die mit einer Gaseinleitung über eine Fritte am Boden des Reaktionsgefässes und einer UV-Tauchlampe versehen war. Die Tauchlampe wurde von oben in das Gefäss eingeführt, so dass die Zone mit der grössten Lichtintensität möglichst dicht über der Eintrittsöffnung der Gaseinleitung lag, durch die Chlor eingeleitet wurde. Die Chlorierungsapparatur enthielt weiterhin eine Dosiervorrichtung für Flüssigkeiten, die in den Boden des Reaktors führte. Die abziehenden Gase wurden zunächst über einen Kühler geführt, der mit einem Blasenzähler ausgestattet wer und dann in einen mit wässrigem Alkali gefüllten Turm geleitet.

Beispiel 1

In der oben beschriebenen Apparatur wurden 102 g 2,2-Dichlor-1,4-benzodioxen in 350 ml Tetrachlormethan gelöst und bei 79°C unter UV-Bestrahlung bis zur Sättigung Chlor eingeleitet. Nach destillativer Aufarbeitung wurden 118 g, 2,2,3,3-Tetrachlor-1,4-benzodioxen mit einem Siedepunkt von 140–145°C bei 20 mbar und einem Schmelzpunkt von 88–92°C erhalten.

Zu Beginn der Chloreinleitung wurde darauf geachtet, dass die Chlorkonzentration im Reaktionsgemisch 0,5 Gew.-% nicht überstieg.

Beispiel 2

Es wurde gearbeitet wie in Beispiel 1, jedoch wurde nicht mit UV-Licht bestrahlt, sondern stattdessen 2 g Dibenzoylperoxid gelöst in Chloroform zugesetzt. Es wurden 109 g 2,2,3,3-Tetrachlor-1,4-benzodioxen erhalten.

Beispiel 3

Es wurde verfahren wie in Beispiel 1, jedoch wurden 50 g Benzodioxen und 400 ml Tetrachlormethan eingesetzt. Es wurden 73 g 2,2,3,3-Tetrachlor-1,4-benzodioxen erhalten.

Beispiel 4

Es wurde verfahren wie in Beispiel 1, jedoch wurden 50 g 2-Chlorbenzodioxen in 150 ml Phosphoroxychlorid bei 110°C chloriert und 74 g 2,2,3,3-Tetrachlor-1,4-benzodioxen erhalten.

Beispiel 5

In 250 ml Tetrachlormethan wurden 20 g 6-Isocyanato-1,4-benzodioxen unter Rückfluss und UV-Belichtung bis zur Sättigung chloriert. Die destillative Aufarbeitung ergab 18 g 6-Isocyanato-2,2,3,3-Tetrachlor-1,4-benzodioxen mit einem Siedepunkt von 120–128°C bei 20 mbar und einem Brechungsindex von $n_D^{20}$ von 1,5840.

Beispiel 6

In gleicher Weise wie in Beispiel 5 wurden 40 g 6-Phenyl-1,4-benzodioxen in 450 ml Tetrachlormethan chloriert. Es wurden 39 g 6-Phenyl-2,2,3,3-tetrachlor-1,4-benzodioxen mit einem Siedepunkt von 185–190°C bei 0,1 mbar und einem Schmelzpunkt von 115–117°C erhalten.

Beispiel 7

Entsprechend der Arbeitsweise von Beispiel 1 wurden 45 g 6-Chlorcarbonyl-1,4-benzodioxen in 175 ml Phosphoroxychlorid bei 110°C unter Belichtung chloriert. Es wurden 47 g 6-Chlorcarbonyl-2,2,3,3-Tetrachlor-1,4-benzodioxen mit einem Siedepunkt von 142–147°C bei 0,02 mbar erhalten.

Beispiel 8

In der oben beschriebenen Apparatur wurden 205 g 2,2-Dichlor-1,4-benzodioxen in 380 ml Phosphoroxychlorid bei Rückflusstemperatur unter Belichtung bis zu einem Umsatz von über 98% chloriert. Der Umsatz wurde durch gaschromatographische Analyse bestimmt. Durch fraktionierte Destillation wurden 230 g 2,2,3,3-Tetrachlor-1,4-benzodioxen erhalten.

Beispiel 9

Entsprechend der Arbeitsweise von Beispiel 1 wurden 40 g 6-Chlor-1,4-benzodioxen chloriert und 65 g 2,2,3,3,6-Pentachlor-1,4-benzodioxen mit einem Siedepunkt von 130–132°C bei 0,3 mbar erhalten.

**Patentansprüche**

1. Verfahren zur Herstellung von 2,2,3,3-Tetrachlor-1,4-benzodioxenen der Formel

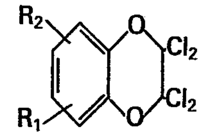

in der

$R_1$ und $R_2$ unabhängig voneinander für H, Cl, F, $CF_3$, $CCl_3$, COCl, CN, $OCOCF_3$, $OCOCCl_3$, $OCOCF_2Cl$, OCOCl, OCOF, NCO, Phenyl oder mit einem oder mehreren der vorstehenden Reste substituiertes Phenyl stehen, dadurch gekennzeichnet, dass man eine oder mehrere Verbindungen der Formel

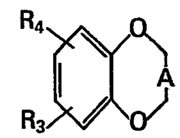

in der

$R_3$ und $R_4$ unabhängig voneinander für H, Cl, F, $CF_3$, $CCl_3$, COCl, CN, $OCOCF_3$, $OCOCCl_3$, $OCOCF_2Cl$, OCOCl, OCOF, NCO, CHO, $CH_3$, Phenyl oder mit einem oder mehreren der vorstehenden Reste substituiertes Phenyl stehen und

A für $CH_2$-$CH_2$, CHCl-$CH_2$, $CCl_2$-$CH_2$, CHCl-CHCl, $CCl_2$-CHCl, CH=CH, CCl=CH oder CCl=CCl steht, mit Chlor in Gegenwart eines Radikalbildners umsetzt.

2. Verfahren nach Anspruch 1, dadurch gekennzeichnet, dass man Verbindungen der angegebenen Formel einsetzt, bei denen $R_3$ und $R_4$ unabhängig voneinander für H, Cl, F, COCl, CN, $CH_3$ oder CHO stehen.

3. Verfahren nach Anspruch 1, dadurch gekennzeichnet, dass man Verbindungen der angegebenen Formel einsetzt, bei denen A für $CH_2$–$CH_2$, $CCl_2$-$CH_2$, CHCl–CHCl oder CH=CH steht.

4. Verfahren nach Ansprüchen 1 bis 3, dadurch gekennzeichnet, dass man pro Mol einzuführendes Chlor 1 bis 5 Mole $Cl_2$ und zusätzlich pro Mol abzuspaltenden Wasserstoff, falls solcher vorhanden ist, weitere 1 bis 5 Mole $Cl_2$ einsetzt.

5. Verfahren nach Ansprüchen 1 bis 4, dadurch gekennzeichnet, dass man beim Einsatz von Verbindungen der angegebenen Formel, bei denen A für $CH_2$–$CH_2$ oder CH=CH steht, die Chlorkonzentration im Reaktionsgemisch unter 0,5 bis 1 Gew.-% hält, bis sich in den Fällen, in den A für $CH_2$-$CH_2$ steht das entsprechende Monochlor-1,4-benzodioxen und in den Fällen, in denen A für CH=CH steht, das entsprechende Dichlor-1,4-benzodioxen gebildet hat.

6. Verfahren nach Ansprüchen 1 bis 5, dadurch gekennzeichnet, dass man als Radikalbildner UV-Licht, Azoisobuttersäurenitril oder Benzoylperoxid einsetzt.

7. Verfahren nach Ansprüchen 1 bis 6, dadurch gekennzeichnet, dass man die Umsetzung in Gegenwart eines Lösungsmittels durchführt.

8. Verfahren nach Ansprüchen 1 bis 7, dadurch gekennzeichnet, dass man die Umsetzung bei Temperaturen von 25 bis 180°C durchführt.

9. Verfahren nach Anspruch 7, dadurch gekennzeichnet, dass man die Umsetzung am Siedepunkt des Lösungsmittels durchführt.

10. 6-Isocyanato-2,2,3,3-Tetrachlor-1,4-benzodioxen der Formel

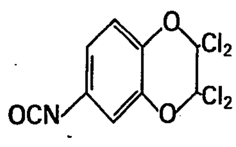

**Claims**

1. Process for the preparation of 2,2,3,3-tetrachloro-1,4-benzodioxenes of the formula

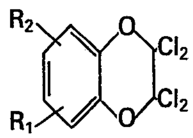

in which

$R_1$ and $R_2$ independently of one another represent H, Cl, F, $CF_3$, $CCl_3$, COCl, CN, $OCOCF_3$, $OCOCCl_3$, $OCOCF_2Cl$, OCOCl, OCOF, NCO, phenyl or phenyl which is substituted by one or more of the above radicals, characterized in that one or more compounds of the formula

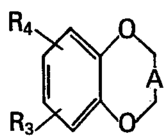

in which

$R_3$ and $R_4$ independently of one another represent H, Cl, F, $CF_3$, $CCl_3$, COCl, CN, $OCOCF_3$, $OCOCCl_3$, $OCOCF_2Cl$, OCOCl, OCOF, NCO, CHO, $CH_3$, phenyl or phenyl which is substituted by one or more of the above radicals and

A represents $CH_2$-$CH_2$, CHCl-$CH_2$, $CCl_2$-$CH_2$, CHCl-CHCl, $CCl_2$-CHCl, CH=CH, CCl=CH or CCl=CCl, are reacted with chlorine in the presence of an agent which forms free radicals.

2. Process according to Claim 1, characterized in that compounds of the given formula in which $R_3$ and $R_4$ independently of one another represent H, Cl, F, COCl, CN, $CH_3$ or CHO are employed.

3. Process according to Claim 1, characterized in that compounds of the given formula in which A represents $CH_2$-$CH_2$, $CCl_2$-$CH_2$, CHVI-CHCl or CH=CH are employed.

4. Process according to Claims 1 to 3, characterized in that 1 to 5 moles of $Cl_2$ are employed per mole of chlorine to be introduced, and in addition a further 1 to 5 moles of $Cl_2$ are employed per mole of hydrogen to be split off, if such hydrogen is present.

5. Process according to Claims 1 to 4, characterized in that, if compounds of the given formula in which A represents $CH_2$-$CH_2$ or CH=CH are employed, the chlorine concentration in the reaction mixture is kept below 0.5 to 1% by weight, until, in cases where A represents $CH_2$-$CH_2$, the corresponding monochloro-1,4-benzodioxene has formed, and in cases where A represents CH=CH, the corresponding dichloro-1,4-benzodioxene has formed.

6. Process according to Claims 1 to 5, characterized in that UV light, azoisobutyronitrile or benzoyl peroxide is used as the agent which forms free radicals.

7. Process according to Claims 1 to 6, characterized in that the reaction is carried out in the presence of a solvent.

8. Process according to Claims 1 to 7, characterized in that the reaction is carried out at temperatures from 25 to 180°C.

9. Process according to Claim 7, characterized in that the reaction is carried out at the boiling point of the solvent.

10. 6-Isocyanato-2,2,3,3-tetrachloro-1,4-benzodioxene of the formula

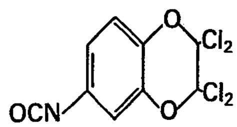

**Revendications**

1. Procédé de production de 2,2,3,3-tétrachloro-1,4-benzodioxènes de formule

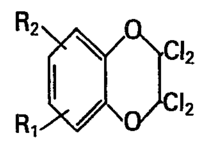

dans laquelle

$R_1$ et $R_2$ représentent, indépendamment l'un de l'autre, H, Cl, F, $CF_3$, $CCl_3$, COCl, CN, $OCOCF_3$, $OCOCCl_3$, $OCOCF_2Cl$, OCOCl, OCOF, NCO, phényle ou un groupe phényle substitué avec un ou plusieurs des restes ci-dessus, caractérisé en ce qu'on fait réagir avec du chlore en présence d'un générateur de radicaux un ou plusieurs composés de formule

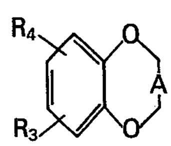

dans laquelle

$R_3$ et $R_4$, indépendamment l'un de l'autre, représentent H, Cl, F, $CF_3$, $CCl_3$, COCl, CN, $OCOCF_3$, $OCOCl_3$, $OCOCF_2Cl$, OCOCl, OCOF, NCO, CHO, $CH_3$, phényle ou un groupe phényle substitué avec un ou plusieurs des restes ci-dessus et

A est un groupe $CH_2$-$CH_2$, CHCl-$CH_2$, $CCl_2$-$CH_2$, CHCl-CHCl, $CCl_2$-CHCl, CH-CH, CCl=CH ou CCl=CCl.

2. Procédé suivant la revendication 1, caractérisé en ce qu'on utilise des composés de la formule indiquée dans lesquels $R_3$ et $R_4$ représentent, indépendamment l'un de l'autre, H, Cl, F, COCl, CN, $CH_3$ ou CHO.

3. Procédé suivant la revendication 1, caractérisé en ce qu'on utilise des composés de la formule indiquée dans lesquels A représente $CH_2$-$CH_2$, $CCl_2$-$CH_2$, CHCl-CHCl ou CH=CH.

4. Procédé suivant les revendications 1 à 3, caractérisé en ce qu'on utilise par mole de chlore à introduire 1 à 5 moles de $Cl_2$ et en outre, par mole d'hydrogène à éliminer, au cas où un tel hydrogène est présent, 1 à 5 moles supplémentaires de $Cl_2$.

5. Procédé suivant les revendications 1 à 4, caractérisé en ce que dans le cas de l'utilisation de composés de la formule indiquée dans lesquels A représente $CH_2$-$CH_2$ ou CH=CH, on maintient la concentration en chlore dans le mélange réactionnel en dessous de 0,1 à 1% en poids jusqu'à ce que se soient formés le monochloro-1,4-benzodioxène correspondant dans les cas dans lesquels A représente $CH_2$-$CH_2$, et le dichloro-1,4-benzodioxène correspondant dans les cas dans lesquels A représente CH=CH.

6. Procédé suivant les revendications 1 à 5, caractérisé en ce qu'on utilise comme générateurs de radicaux la lumière ultraviolette, l'azoisobutyronitrile ou le peroxyde de benzoyle.

7. Procédé suivant les revendications 1 à 6 caractérisé en ce qu'on conduit la réaction en présence d'un solvant.

8. Procédé suivant les revendications 1 à 7, caractérisé en ce qu'on conduit la réaction à des températures de 25 à 180°C.

9. Procédé suivant la revendication 7, caractérisé en ce qu'on conduit la réaction au point d'ébullition du solvant.

10. Le 6-isocyanato-2,2,3,3-tétrachloro-1,4-benzodioxène de formule